# EUROPEAN PATENT APPLICATION

(11) **EP 4 527 298 A1**
(43) Date of publication of application: **26.03.2025**
(21) Application number: 24201626.9
(22) Date of filing: 20.09.2024
(51) Int. Cl.: A61B 6/00, A61B 6/03, A61B 6/46, A61B 6/50

(54) **X-RAY DOSE ADJUSTMENT METHOD AND APPARATUS, AND MEDICAL IMAGING SYSTEM**

(30) Priority: 25.09.2023 CN 202311246162
(71) Applicant: Siemens Healthineers AG, 91301 Forchheim (DE)
(72) Inventor: NI, Bei, Shanghai, 201318 (CN); WANG, Yu Dan, Shanghai, 200092 (CN)
(74) Representative: Horn Kleimann Waitzhofer Schmid-Dreyer Patent- und Rechtsanwälte PartG mbB

(57) **Abstract**

The present disclosure provides an X-ray dose adjustment method and apparatus, and a medical imaging system. The X-ray dose adjustment method for a medical imaging system comprises: acquiring a first image which is captured by the medical imaging system and comprises a region to be examined; subjecting the first image to image segmentation on the basis of a grayscale value of the first image to acquire a first sub-region and a second sub-region, wherein a grayscale value of the first sub-region is greater than a grayscale value of the second sub-region; determining at least one of the first sub-region and the second sub-region to be a target region on the basis of a comparison of the grayscale value of the first sub-region with a first grayscale threshold; and performing dose adjustment on the basis of the target region.

## Description

### TECHNICAL FIELD

The present disclosure relates to the technical field of medical equipment, in particular to an X-ray dose adjustment method and apparatus for a medical imaging system, a medical imaging system, a non-transitory computer-readable storage medium and a computer program product.

### BACKGROUND ART

Medical imaging systems (e.g. computerized tomography systems, digital subtraction angiography systems, etc.) are able to non-invasively acquire and process images of tissues inside the human body. Some medical imaging systems are able to use an emitted X-ray beam to scan an examination subject and thereby acquire information, which is then processed by computer to obtain a reconstructed image. During scanning, the X-ray dose needs to be controlled, to improve the quality of the reconstructed image acquired.

The method described in this section is not necessarily a previously envisaged or used method. Unless otherwise stated, it should not be assumed that any method described in this section is regarded as prior art simply because it is included in this section. Similarly, unless otherwise stated, problems mentioned in this section should not be regarded as having been generally acknowledged in any prior art.

### SUMMARY OF THE INVENTION

According to one aspect of embodiments of the present disclosure, the following are provided: an X-ray dose adjustment method and apparatus for a medical imaging system, a medical imaging system, a non-transitory computer-readable storage medium and a computer program product.

According to one aspect of embodiments of the present disclosure, an X-ray dose adjustment method for a medical imaging system is provided, the method comprising: acquiring a first image which is captured by the medical imaging system and comprises a region to be examined; subjecting the first image to image segmentation on the basis of a grayscale value of the first image to acquire a first sub-region and a second sub-region, wherein a grayscale value of the first sub-region is greater than a grayscale value of the second sub-region; determining at least one of the first sub-region and the second sub-region to be a target region on the basis of a comparison of the grayscale value of the first sub-region with a first grayscale threshold; and performing dose adjustment on the basis of the target region.

According to another aspect of embodiments of the present disclosure, a dose adjustment apparatus for a medical imaging system is provided, the apparatus comprising: a first image acquisition unit, configured to acquire a first image which is captured by the medical imaging system and comprises a region to be examined; an image segmentation unit, configured to subject the first image to image segmentation on the basis of a grayscale value of the first image to acquire a first sub-region and a second sub-region, wherein a grayscale value of the first sub-region is greater than a grayscale value of the second sub-region; a target region determining unit, configured to determine at least one of the first sub-region and the second sub-region to be a target region on the basis of a comparison of the grayscale value of the first sub-region with a first grayscale threshold; and an exposure control unit, configured to perform dose adjustment on the basis of the target region.

According to another aspect of embodiments of the present disclosure, a medical imaging system is provided, comprising: at least one processor; and a memory in communicative connection with the at least one processor, wherein the memory stores a computer program which, when executed by the at least one processor, realizes the method described above.

According to another aspect of embodiments of the present disclosure, a non-transitory computer-readable storage medium storing a computer program is provided, wherein the computer program, when executed by a processor, realizes the method described above.

According to another aspect of embodiments of the present disclosure, a computer program product is provided, comprising a computer program which, when executed by a processor, realizes the method described above.

It should be understood that the content described in this section is not intended to identify key or important features of embodiments of the present disclosure, and not intended to limit the scope of the present disclosure. Other features of the present disclosure will become easy to understand through the following description.

### BRIEF DESCRIPTION OF THE DRAWINGS

The drawings show embodiments demonstratively and form part of this specification, being used together with the textual description of this specification to explain exemplary ways of implementing embodiments. The embodiments shown merely serve an illustrative purpose, and do not limit the scope of the claims. In all of the drawings, identical reference labels denote similar but not necessarily identical key elements.

Embodiments of the present disclosure are described in detail below with reference to the accompanying drawings, to give those skilled in the art a clearer understanding of the abovementioned and other features and advantages of the present disclosure. In the drawings:
Fig. 1 shows a flow chart of an X-ray dose adjustment method for a medical imaging system according to an embodiment of the present disclosure.
Fig. 2 shows a flow chart of part of the process of the X-ray dose adjustment method for a medical imaging system according to an embodiment of the present disclosure.
Fig. 3 shows another flow chart of part of the process of the X-ray dose adjustment method for a medical imaging system according to an embodiment of the present disclosure.
Fig. 4 shows another flow chart of part of the process of the X-ray dose adjustment method for a medical imaging system according to an embodiment of the present disclosure.
Fig. 5 shows another flow chart of part of the process of the X-ray dose adjustment method for a medical imaging system according to an embodiment of the present disclosure.
Fig. 6 shows another flow chart of an X-ray dose adjustment method for a medical imaging system according to an embodiment of the present disclosure.
Fig. 7 shows a structural block diagram of an X-ray dose adjustment apparatus for a medical imaging system according to an embodiment of the present disclosure.
Fig. 8 is a block diagram showing an exemplary electronic device applicable to an exemplary embodiment.

### DETAILED DESCRIPTION OF THE INVENTION

Demonstrative embodiments of the present disclosure are described below with reference to the drawings, including various details of embodiments of the present disclosure to assist understanding, but these should be regarded as merely demonstrative. Thus, those skilled in the art should recognize that various changes and modifications could be made to the embodiments described here without deviating from the scope of the present disclosure. Similarly, for clarity and conciseness, descriptions of commonly known functions and structures are omitted in the descriptions below.

In the present disclosure, unless otherwise stated, the use of the terms "first", "second" etc. to describe various key elements is not intended to define a positional relationship, time sequence relationship or importance relationship of these key elements; such terms are merely used to distinguish one element from another. In some examples, a first key element and a second key element may refer to the same instance of the element in question, whereas in some cases, based on the description in the context, they may also denote different instances.

Terms used in the descriptions of the various examples in the present disclosure are merely intended to describe specific examples, and are not intended to impose restrictions. Unless clearly indicated otherwise in the context, if the quantity of a key element is not specifically defined, the key element may be one or more. In addition, the term "and/or" used herein encompasses any one of the listed items and all possible combinations.

As stated above, during scanning by certain medical imaging systems, the X-ray dose needs to be controlled, to improve the quality of the reconstructed image acquired. In the related art, X-ray exposure parameters (e.g. tube voltage, tube current, exposure time) for imaging a subsequent image frame may be adjusted according to an X-ray exposure dose indicated by the previous image frame acquired by the medical imaging system. For example, one or more region of interest (ROI) may be preset in an imaging region within an organ program (OGP) of the medical imaging system, and an X-ray exposure dose in the one or more ROI may be acquired in order to adjust a subsequent X-ray exposure dose. Generally, one or more preset ROI may be a rectangle located at a fixed position, or may be the entire imaging field of view (FOV) region. However, in the process of operating the medical imaging system to perform imaging, an operator might be unable to modify or reset one or more preset ROI, and as a result, the quality of the image finally acquired might not be what the user was expecting. For example, if the preset ROI is a rectangular region located at the center of the image, and the examination subject is possibly small and only occupies a very small part of the preset ROI, then adjustment of X-ray exposure parameters according to the whole ROI will be unable to correctly reflect the X-ray dose actually received by the examination subject; as a result, it will not be possible to accurately adjust a subsequent X-ray exposure amount, and so good image quality will be unachievable. Similarly, if the preset ROI is the entire imaging FOV region, then it will likewise not be possible to accurately adjust a subsequent X-ray exposure amount if the examination subject occupancy ratio is relatively small.

In view of the above, the present disclosure provides an X-ray dose adjustment method and apparatus for a medical imaging system, a medical imaging system, a non-transitory computer-readable storage medium and a computer program product, which are able to improve X-ray dose adjustment precision, and thereby improve the imaging quality of the medical imaging system.

Embodiments of the present disclosure are described in detail below with reference to the drawings.

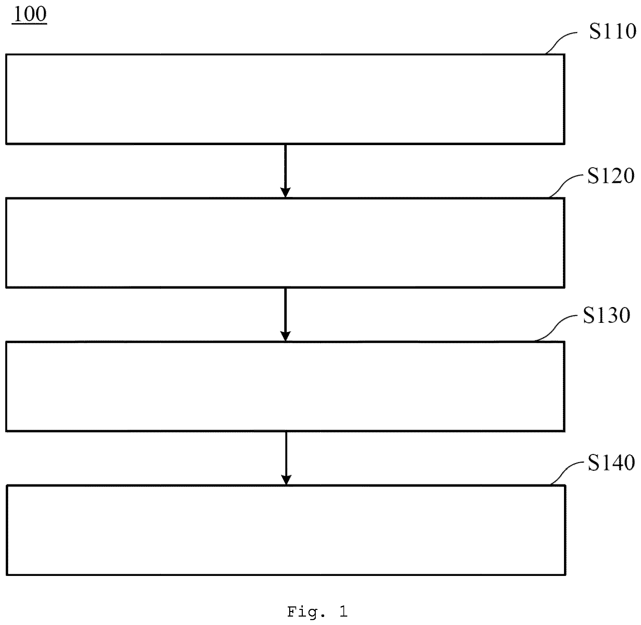

Fig. 1 shows a flow chart of an X-ray dose adjustment method 100 for a medical imaging system according to an embodiment of the present disclosure. As shown in Fig. 1, the method 100 comprises:
step S110, acquiring a first image which is captured by the medical imaging system and comprises a region to be examined;
step S120, based on grayscale values of the first image, subjecting the first image to image segmentation to acquire a first sub-region and a second sub-region, wherein a grayscale value of the first sub-region is greater than a grayscale value of the second sub-region;
step S130, based on a comparison of the grayscale value of the first sub-region with a first grayscale threshold, determining at least one of the first sub-region and the second sub-region to be a target region; and
step S140, based on the target region, performing dose adjustment.

In an example, the medical imaging system may comprise an X-ray generator and an X-ray detector. The X-ray generator may comprise a high-voltage generator and an X-ray tube. In an example, the high-voltage generator can generate a high voltage and supply a high voltage (tube voltage) to two ends of the X-ray tube, and the X-ray tube can generate X-rays under the action of the high voltage. An X-ray detector may be arranged in a ray emission direction of the X-ray generator, the X-ray detector comprising at least one region, which is used for detecting X-rays and may convert a received X-ray amount to a corresponding electrical signal. The medical imaging system may further comprise a measurement imaging unit, which generates an image on the basis of the electrical signal resulting from conversion in the X-ray detector. The region to be examined (e.g. a limb region, such as a hand, chest, shoulder, leg or foot) may be positioned between the X-ray generator and the X-ray detector, and X-rays emitted by the X-ray generator may pass through the region to be examined and reach the X-ray detector.

In step S110, an original image, captured by the medical imaging system and comprising a region to be examined, may be acquired. Correspondingly, the first image may be obtained by processing the original image. For example, the original image may be subjected to filtering, cropping or other image processing to obtain the first image.

When X-rays irradiate the region to be examined, the intensities of X-rays absorbed and scattered by different regions are different, so an image with different degrees of brightness is formed on the detector. Specifically, when a beam of X-rays of uniform intensity irradiates the human body, the X-rays will be absorbed and scattered by organs with different tissues, such that the intensity distribution of X-rays passing through the human body changes. These X-rays passing through the human body will be received by the detector and converted to electrical signals, then processed by the measurement imaging unit, to finally form a grayscale image which can be displayed on a screen. Correspondingly, the first image may be a grayscale image.

In step S120, based on grayscale values of the first image, the first image is subjected to image segmentation to acquire a first sub-region and a second sub-region. In an example, a thresholding algorithm may be used to divide the first image into a first sub-region and a second sub-region according to grayscale values of pixels of the first image. For example, a global threshold or a local threshold may be chosen to segment the image. In addition, an image segmentation algorithm based on clustering (e.g. K-means, GMM algorithm, etc.) may be used to divide the first image into a first sub-region and a second sub-region. For example, pixels may be clustered according to their grayscale value and spatial position characteristics to segment the image.

In the process of performing an X-ray examination of an animal body or human body, bony and non-bony regions for example in the acquired image will appear with different degrees of grayness; generally, the higher density of bone prevents more of the X-ray dose from penetrating, so regions corresponding to bony parts in the image have lower grayscale values (the image is darker), whereas regions corresponding to non-bony parts such as soft tissue in the image might have higher grayscale values (the image is brighter). In step S130, based on a comparison of the grayscale value of the first sub-region with a first grayscale threshold, at least one of the first sub-region and the second sub-region is determined to be a target region. Since the grayscale value of the first sub-region is greater than the grayscale value of the second sub-region, the first sub-region is the brighter of the two regions, and the second sub-region is the darker of the two regions. The grayscale value of the brighter of the two regions is compared with the first grayscale threshold to determine at least one of the first sub-region and the second sub-region to be a target region, which can serve as an ROI.

In step S140, based on the target region determined in step S130, dose adjustment is performed. For example, an X-ray exposure dose requiring to be further increased or decreased may be computed according to a grayscale value of the target region, so as to perform dose adjustment. In an example, dose adjustment may be performed on the basis of an average grayscale value of the target region.

Thus, by means of the above-described embodiments of the present disclosure, based on grayscale values of the first image, the first image is subjected to image segmentation to acquire the first sub-region and the second sub-region, and based on a comparison of the grayscale value of the first sub-region which has the greater grayscale value with the first grayscale threshold, at least one of the first sub-region and the second sub-region is determined to be the target region; the acquired target region is able to match more accurately the region to be examined, i.e. able to reflect more accurately the position of the region to be examined in the image. When the region to be examined changes, the target region also changes accordingly. Correspondingly, dose adjustment is performed on the basis of the target region, and since the target region is able to correctly reflect the X-ray dose actually received by the region to be examined, the precision of X-ray dose adjustment can be increased, thereby improving the imaging quality of the medical imaging system.

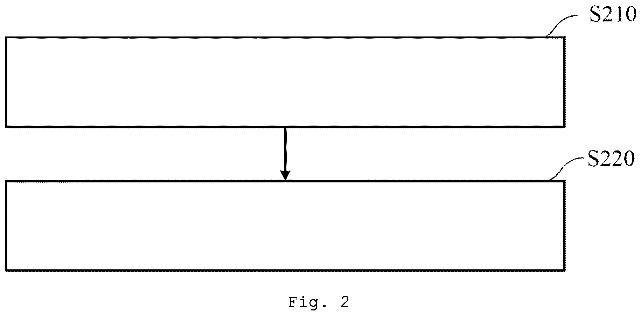

Fig. 2 shows a flow chart of part of the process of the X-ray dose adjustment method 100 for a medical imaging system according to an embodiment of the present disclosure.

According to some embodiments, as shown in Fig. 2, step S130 mentioned above may comprise:
step S210, comparing a first average grayscale value of the first sub-region with a first grayscale threshold; and
step S220, in response to the first average grayscale value being greater than the first grayscale threshold, determining the second sub-region to be the target region. In some examples, the first grayscale threshold may for example be a preset grayscale value; for example, a grayscale value of 200 may serve as the first grayscale threshold. In other examples, the first grayscale threshold may be determined according to the first image; for example, the first grayscale threshold may be 75% - 95% of a maximum grayscale value of the first image.

When the first average grayscale value of the first sub-region is greater than the first grayscale threshold, this indicates that the brightness of the first sub-region is higher than a certain level. Thus, the first sub-region is considered to be a "blank" region (a directly irradiated region) in the image; that is, within the first sub-region, there is no region to be examined (e.g. bone) blocking the passage of X-rays through this region, so the brightness of the first sub-region is higher. In this case, the second sub-region (i.e. the region with the lower grayscale value) is considered to be a region corresponding to the region to be examined (e.g. bone) in the image; in this region, since the region to be examined (e.g. bone) at least partly blocks the passage of X-rays through this region, the brightness of the second sub-region is lower.

Since it is determined that there is no region to be examined in the first sub-region, the second sub-region (i.e. the region with the lower grayscale value) is determined to be the target region, and the position of the region to be examined in the image can thus be reflected accurately. In a scenario in which the region to be examined is small relative to the imaging FOV region as a whole, most of the imaging FOV region as a whole has relatively high brightness due to not being blocked by the region to be examined. In such a scenario, the first sub-region representing a "blank" region will not be determined to be the target region; the second sub-region representing the region to be examined will instead be determined to be target region.

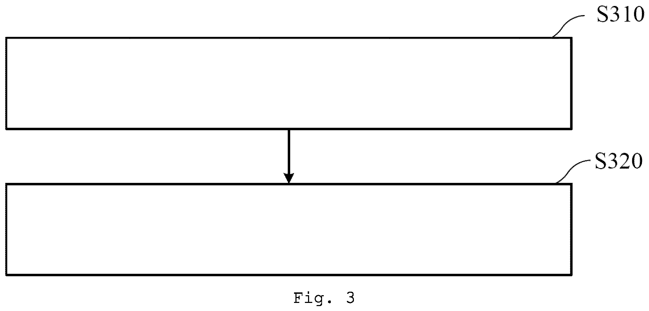

Fig. 3 shows a flow chart of part of the process of the X-ray dose adjustment method 100 for a medical imaging system according to an embodiment of the present disclosure.

According to some embodiments, step S130 mentioned above may comprise:
step S310, comparing a first average grayscale value of the first sub-region with a first grayscale threshold; and
step S320, in response to the first average grayscale value being less than the first grayscale threshold, determining both the first sub-region and the second sub-region to be the target region.

In some examples, the first grayscale threshold may for example be a preset grayscale value; for example, a grayscale value of 200 may serve as the first grayscale threshold. In other examples, the first grayscale threshold may be determined according to the first image; for example, the first grayscale threshold may be 75% - 95% of a maximum grayscale value of the first image.

When the first average grayscale value of the first sub-region is less than the first grayscale threshold, this indicates that the brightness of the first sub-region is lower than a certain level. Thus, in this case, the second sub-region (i.e. the region with the lower grayscale value) is considered to be a region corresponding to the region to be examined (e.g. bone) in the image; in this region, since the region to be examined (e.g. bone) at least partly blocks the passage of X-rays through this region, the brightness of the second sub-region is lower. In addition, the first sub-region is considered to be a "non-blank" region in the image; that is, within the first sub-region, there might be a region to be examined or peripheral tissue (such as connective tissue or muscle) or a peripheral region of a region to be examined that at least partly blocks the passage of X-rays through this region, so the brightness of the first sub-region is slightly lower (but higher than the brightness of the second sub-region).

Since it is determined that the first sub-region might contain a region (such as connective tissue or muscle) related to the region to be examined, both the first sub-region and the second sub-region are determined to be the target region, and the position of the region to be examined in the image can thus be reflected accurately. In a scenario in which the region to be examined is large relative to the imaging FOV region as a whole, most of the imaging FOV region as a whole has relatively low brightness due to being blocked by the region to be examined or a region related to the region to be examined. In such a scenario, the second sub-region representing the region to be examined (e.g. bone) and the region (such as connective tissue or muscle) related to the region to be examined are both determined to be the target region.

According to some embodiments, the method 100 may further comprise acquiring the maximum grayscale value in the first image. Furthermore, the first grayscale threshold may be set within the range of 75% - 95% of the maximum grayscale value; for example, the first grayscale threshold may be set within the range of 80% - 90% of the maximum grayscale value.

Thus, as the first grayscale threshold is set on the basis of the maximum grayscale value in the first image, the first grayscale threshold can be adjusted according to the actual characteristics of the first image, so that the first grayscale threshold reflects the maximum brightness characteristic of the first image more accurately, and it is thus possible to further improve the rationality and accuracy with which the first sub-region and the second sub-region are distinguished from one another. Thus, it is possible to further improve the precision of X-ray dose adjustment, so as to further improve the imaging quality of the medical imaging system.

According to some embodiments, step S120 mentioned above may comprise: based on grayscale values of the first image, determining a second grayscale threshold. In the first image, pixels with grayscale values greater than the second grayscale threshold are segmented into a first sub-region, pixels with grayscale values less than the second grayscale threshold are segmented into a second sub-region, and the determined second grayscale threshold is such that the variance between a first average grayscale value of the first sub-region and a second average grayscale value of the second sub-region is maximized.

In an example, a maximum inter-class variance algorithm (OTSU algorithm) may be used to determine, on the basis of grayscale values of the first image, a second grayscale threshold for distinguishing a first sub-region from a second sub-region. The variance is a metric for uniformity of grayscale distribution; therefore, if the inter-class variance between the first sub-region and the second sub-region is greater, this indicates that the difference between the two parts forming the image is greater; if a pixel which should have been segmented to the first sub-region is "erroneously" segmented to the second sub-region, the inter-class variance will decrease, and vice versa. Thus, image segmentation that maximizes the inter-class variance means that the probability of error is minimized. Thus, a second grayscale threshold capable of better distinguishing two sub-regions may be computed simply and quickly, without being influenced by image brightness or contrast.

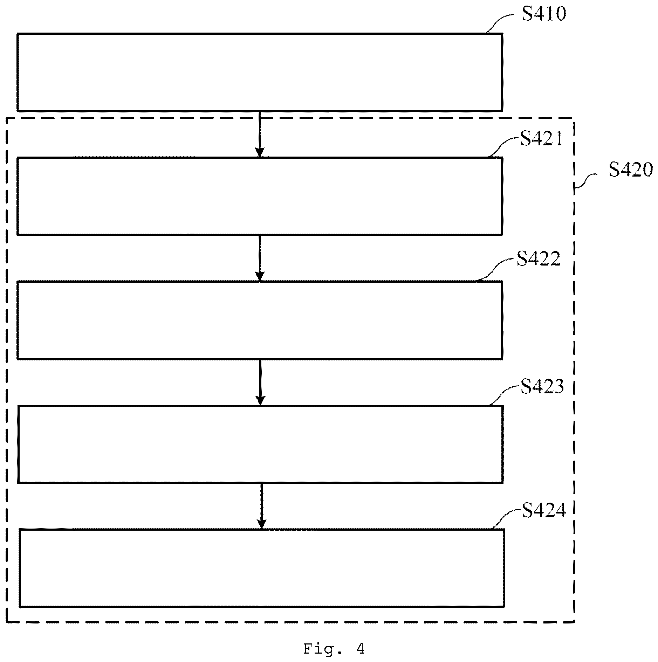

According to some embodiments, the medical imaging system may further comprise a collimator for limiting an X-ray irradiation range. The collimator may for example comprise a filter for limiting the X-ray irradiation range or blocking the passage of X-rays; in an example, a peripheral region of the collimator may comprise lead or tungsten nylon capable of blocking X-rays, and a middle region of the collimator surrounded by the peripheral region can transmit X-rays. Furthermore, referring to Fig. 4, Fig. 4 shows a flow chart of part of the process of the X-ray dose adjustment method 100 for a medical imaging system according to an embodiment of the present disclosure.

Step S110 mentioned above may comprise:
step S410, acquiring an original image captured by the medical imaging system, the original image comprising a region to be examined and a collimator region; and
step S420, removing the collimator region from the original image to acquire a first image.

Since the collimator region can block the passage of X-rays, the collimator region in the original image will have a very low grayscale value (for example, close to 0). By removing the collimator region in the original image, the collimator region can be prevented from being erroneously segmented into the second sub-region in a subsequent step, i.e. the collimator region can be prevented from being erroneously considered to be a region representing a region to be examined, which would result in reduced precision of X-ray dose adjustment.

According to some embodiments, continuing to refer to Fig. 4, step S420 mentioned above may comprise:
step S421, acquiring current coordinates of the collimator in the medical imaging system;
step S422, determining an image contour corresponding to the collimator region in the original image according to the current coordinates of the collimator;
step S423: determining a contour, formed by extension by a predetermined distance from a side of the image contour remote from the collimator region, to be a removal boundary; and
step S424, removing from the original image a region defined outside the removal boundary, so as to acquire a first image.

In step S421, initial coordinates of the collimator in the medical imaging system may be known; when a user operates the collimator to move, the current coordinates of the collimator in the medical imaging system may be determined according to a movement control instruction (e.g. movement speed, movement distance) and on the basis of the initial coordinates of the collimator.

In step S422, an image contour corresponding to the collimator region in the original image may be determined according to a mapping between a collimator coordinate system and a ray detector coordinate system and according to the current coordinates of the collimator; for example, coordinates of pixel points corresponding to an inner peripheral contour of the collimator region may be determined.

In step S423, a contour, formed by extension by a predetermined distance from a side of the image contour remote from the collimator region, is determined to be a removal boundary. In other words, the removal boundary is "contracted" inwards relative to the image contour, and the predetermined distance may be a distance of a number of pixels.

Thus, when the region defined outside the removal boundary is removed from the original image, the collimator region of the original image can be completely removed because the boundary region affords a certain "safety margin", thus further preventing the collimator region from being erroneously considered to be a region representing a region to be examined, which would result in reduced precision of X-ray dose adjustment.

It will be understood that the predetermined distance may be determined according to the particular type of the collimator or the particular type of the region to be examined.

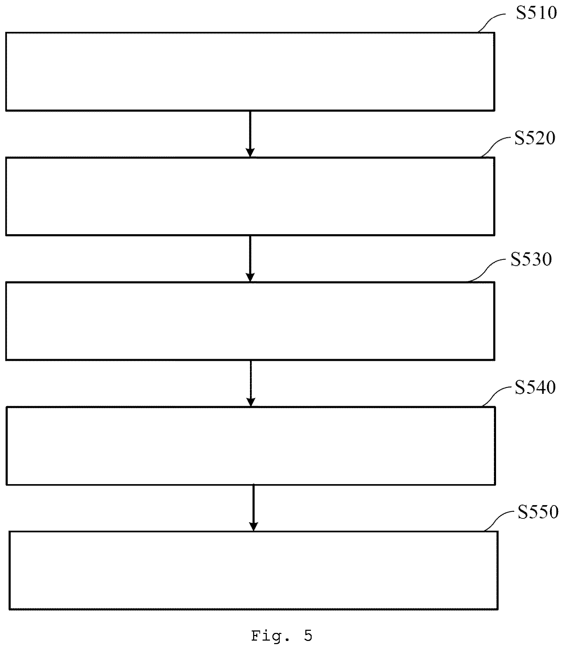

Fig. 5 shows another flow chart of part of the process of the X-ray dose adjustment method 100 for a medical imaging system according to an embodiment of the present disclosure.

According to some embodiments, as shown in Fig. 5, step S110 mentioned above may comprise:
step S510, acquiring an original image captured by the medical imaging system, the original image comprising a region to be examined;
step S520, based on the original image, determining an actual dose produced by X-rays emitted by the medical imaging system;
step S530, comparing the actual dose with a target dose; and
step S540, in response to the difference between the actual dose and the target dose being within a preset range, acquiring a first image on the basis of the original image.

In an example, the actual dose of X-rays emitted by the medical imaging system may be determined according to grayscale values (e.g. an average grayscale value) of the original image. When the difference between the actual dose and the target dose is within the preset range, this indicates that the exposure quality of the original image currently obtained meets a condition; a first image may be acquired on the basis of the original image, and the processing of steps S120 to S140 for example may be further performed on the basis of the first image.

According to some embodiments, step S540 mentioned above may comprise: filtering the original image to acquire a first image with image noise points removed.

For example, the original image may be subjected to Gaussian filtering, median filtering or adaptive filtering, etc. to remove image noise points from the original image. Median filtering removes noise points from an image by sorting the values of neighboring pixels and setting the median value to be the value of the pixel concerned. Gaussian filtering removes noise from an image by using a Gaussian function to subject the image to convolution. Adaptive filtering is a type of filter which adjusts filter weight according to local characteristics of an image; it removes noise from the image by setting the value of each pixel to be a weighted average of neighboring pixel values.

According to some embodiments, further referring to Fig. 5, the method 100 may further comprise:
step S550, in response to the difference between the actual dose and the target dose being greater than a maximum value of the preset range, performing dose adjustment on the basis of the original image. When the difference between the actual dose and the target dose is greater than the maximum value of the preset range, this indicates that the actual dose has exceeded a permitted range (e.g. the X-ray exposure amount is too low or too high); in this case, dose adjustment may be performed on the basis of the current original image without performing subsequent steps S120 to S 140, so as to acquire a new original image again. Thus, the efficiency of dose adjustment can be increased, preventing extreme situations from interfering with the process of dose adjustment.

According to some embodiments, step S140 mentioned above may comprise:
based on the target region, determining a deviation between an actual dose of X-rays emitted by the medical imaging system and a target dose; and
based on the deviation, computing an updated ray exposure parameter for exposure of the next image.

In an example, updated exposure parameters such as tube voltage tube current and exposure time may be computed, and ray exposure of the next image frame is controlled on the basis of the updated ray exposure parameters.

The technical solution of the present disclosure is explained further below by means of a particular embodiment.

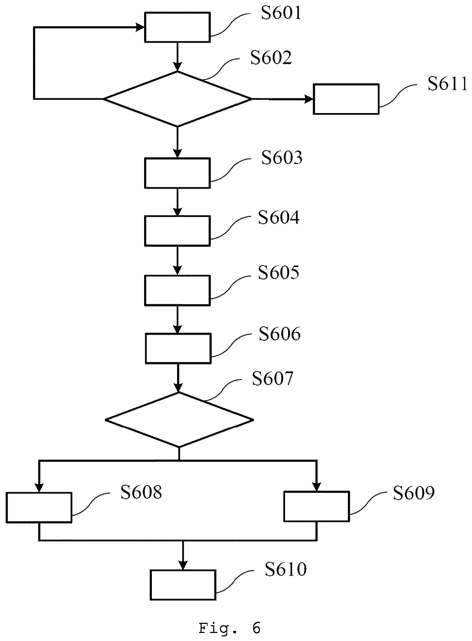

Referring to Fig. 6, Fig. 6 shows a flow chart of an X-ray dose adjustment method 200 for a medical imaging system according to an embodiment of the present disclosure.

In step S601, an original image captured by the medical imaging system is acquired, the original image comprising a region to be examined and a collimator region.

In step S602, based on the original image, an actual dose of X-rays emitted by the medical imaging system is determined, e.g. the actual dose of X-rays emitted by the medical imaging system when capturing the original image is determined according to a relationship between X-ray dose and grayscale values or signal-to-noise ratio, etc. of the original image; and the actual dose is compared with a target dose. When it is determined that the difference between the actual dose and the target dose is within a preset range, the method proceeds to step S603, wherein a minimum value of the preset range is a maximum permitted difference between the actual dose and the target dose or is less than the maximum permitted difference, and a maximum value of the preset range is an upper limit value at which image segmentation can be realized, and if the difference is greater than the maximum value of the preset range, the image quality is not suitable for performing image segmentation; when it is determined that the difference between the actual dose and the target dose is greater than the maximum value of the preset range, the method returns to step S601, i.e. based on the current original image, dose adjustment is performed according to a preset region, such as an ROI preset by an OGP or an entire imaging FOV region or a region formed by excluding a collimator region from the ROI or the entire imaging FOV region, so as to re-acquire an original image; when it is determined that the difference between the actual dose and the target dose is less than the minimum value of the preset range, i.e. the difference between the actual dose and the target dose is within a permitted range, step S611 is performed, to complete the dose adjustment, and a fluoroscopy or exposure operation can be started.

In step S603, the original image is filtered to remove noise points from the original image.

In step S604, a collimator region is removed from the image from which noise points have been removed; specifically, current coordinates of a collimator in the medical imaging system are acquired; an image contour corresponding to a collimator region in the original image is determined according to the current coordinates of the collimator; a contour, formed by extension by a predetermined distance from a side of the image contour remote from the collimator region, is determined to be a removal boundary; and a region defined outside the removal boundary is removed from the image, to acquire a first image.

In step S605, a maximum grayscale value in the first image is determined.

In step S606, based on grayscale values of the first image, a second grayscale threshold is determined. In the first image, pixels with grayscale values greater than the second grayscale threshold are segmented into a first sub-region, pixels with grayscale values less than the second grayscale threshold are segmented into a second sub-region, and the second grayscale threshold is such that the variance between a first average grayscale value of the first sub-region and a second average grayscale value of the second sub-region is maximized.

In step S607, an average grayscale value of the first sub-region is compared with a first grayscale threshold. The first grayscale threshold is within the range of 80% - 90% of the maximum grayscale value determined in step S605. When it is determined that the first average grayscale value is greater than the first grayscale threshold, the second sub-region is determined to be a target region, i.e. step S608 is performed; when it is determined that the first average grayscale value is less than the first grayscale threshold, both the first sub-region and the second sub-region are determined to be a target region, i.e. step S609 is performed.

In step S610, based on the target region determined in step S608 or S609, dose adjustment is performed. Specifically, based on a relationship between dose and grayscale values or signal-to-noise ratio, etc. of the target region, an actual dose during capture of the original image by the medical imaging system is determined, the actual dose is compared with the target dose; and exposure parameters of the medical imaging system, such as tube voltage, tube current and exposure time of an X-ray generator, are adjusted according to a comparison result.

According to another aspect of the present disclosure, a dose adjustment apparatus for a medical imaging system is provided.

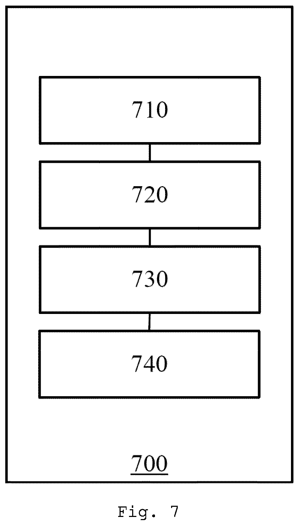

Fig. 7 shows a structural block diagram of an X-ray dose adjustment apparatus 700 for a medical imaging system according to an embodiment of the present disclosure. As shown in Fig. 7, the apparatus 700 comprises:
a first image acquisition unit 710, configured to acquire a first image which is captured by the medical imaging system and comprises a region to be examined;
an image segmentation unit 720, configured to subject the first image to image segmentation on the basis of grayscale values of the first image to acquire a first sub-region and a second sub-region, wherein a grayscale value of the first sub-region is greater than a grayscale value of the second sub-region;
a target region determining unit 730, configured to determine at least one of the first sub-region and the second sub-region to be a target region on the basis of a comparison of the grayscale value of the first sub-region with a first grayscale threshold; and
an exposure control unit 740, configured to perform dose adjustment on the basis of the target region.

It should be understood that the units of the apparatus 700 shown in Fig. 7 may correspond to the steps in the method 100 described with reference to Fig. 1. Thus, the operations, features and advantages described above in relation to the method 100 likewise apply to the apparatus 700 and the units comprised therein. For conciseness, some operations, features and advantages are not described again here.

According to another aspect of the present disclosure, a medical imaging system is provided, comprising: at least one processor; and a memory in communicative connection with the at least one processor, wherein the memory stores a computer program which, when executed by the at least one processor, realizes the method 100.

According to another aspect of the present disclosure, a non-transitory computer-readable storage medium storing a computer program is provided, wherein the computer program, when executed by a processor, realizes the method 100.

According to another aspect of the present disclosure, a computer program product is provided, comprising a computer program which, when executed by a processor, realizes the method 100.

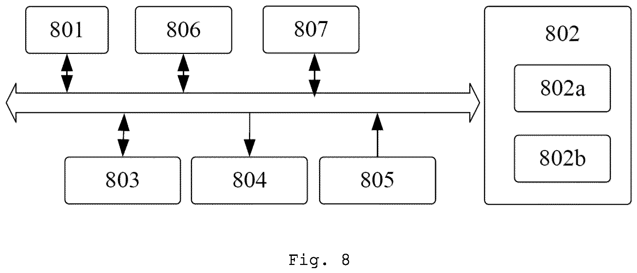

Fig. 8 is a block diagram showing an example of an electronic device 800 according to an exemplary embodiment of the present disclosure. It must be explained that the structure shown in Fig. 8 is merely an example; depending on the specific manner of implementation, the electronic device of the present disclosure may only comprise one or more of the constituent parts shown in Fig. 8. The medical imaging system according to the present disclosure may comprise the electronic device mentioned above.

The electronic device 800 may for example be a general-purpose computer (e.g. a laptop computer, a tablet computer, or various other types of computer), a mobile phone, or a personal digital assistant. According to some embodiments, the electronic device 800 may be a cloud computing device and a smart device. According to some embodiments, the electronic device 800 may be a medical imaging system (e.g. a computerized tomography system, digital subtraction angiography system, etc.).

According to some embodiments, the electronic device 800 may be configured to subject an image, etc. to processing, and transmit a result of the processing to an output device, so that it is provided to a user. The output device may for example be a display screen, a device comprising a display screen, or another output device. For example, the electronic device 800 may be configured to subject the image to target detection, and transmit a result of target detection to a display device for display; the electronic device 800 may also be configured to subject the image to enhancement processing, and transmit a result of enhancement to a display device for display.

The electronic device 800 may comprise an image processing circuit 803; the image processing circuit 803 may be configured to subject the image to various types of image processing. The image processing circuit 803 may for example be configured to subject the image to at least one of the following types of image processing: noise reduction, geometric correction, feature extraction, detection and/or identification of objects in the image, and enhancement processing. The image processing circuit 803 may use custom hardware, and/or may be realized using hardware, software, firmware, middleware, microcode, hardware description language or any combination thereof. For example, one or more of the various circuits mentioned above may be realized by using assembly language or hardware programming language (such as VERILOG, VHDL, C++) to program hardware (e.g. a programmable logic circuit comprising a field programmable gate array (FPGA) and/or a programmable logic array (PLA)), using logic and algorithms according to the present disclosure.

According to some embodiments, the electronic device 800 may further comprise an output device 804; the output device 804 may be any type of device used to present information, and may include, but is not limited to, a display screen, a terminal with display functionality, earphones, a loudspeaker, a vibrator and/or a printer, etc.

According to some embodiments, the electronic device 800 may further comprise an input device 805; the input device 805 may be any type of device for inputting information to the electronic device 800, and may include, but is not limited to, various types of sensor, mouse, keyboard, touch screen, push button, control stick, microphone and/or remote controller, etc.

According to some embodiments, the electronic device 800 may further comprise a communication device 806; the communication device 806 may be any type of device or system enabling communication with an external device and/or with a network, and may include, but is not limited to, a modem, a network card, an infrared communication device, a wireless communication device and/or a chipset, e.g. a Bluetooth device, a 802.11 device, a WiFi device, a WiMax device, a cellular communication device and/or similar.

According to some embodiments, the electronic device 800 may further comprise a processor 801. The processor 801 may be any type of processor, and may include, but is not limited to, one or more general-purpose processor and/or one or more dedicated processor (e.g. special processing chip). The processor 801 may for example be, but is not limited to being, a central processing unit (CPU), a graphics processing unit (CPU), or various types of dedicated artificial intelligence (AI) computing chips, etc. In an example in which the electronic device 800 may be a magnetic resonance scanning imaging device, the processor 801 may be a processor of a master control computer of the magnetic resonance scanning imaging device.

The electronic device 800 may further comprise a working memory 802 and a storage device 807. The processor 801 may be configured to be able to acquire and execute computer-readable instructions stored in the working memory 802, the storage device 807 or another computer-readable medium, such as program code of an operating system 802a, program code of an application program 802b, etc. The working memory 802 and storage device 807 are examples of computer-readable storage media used to store instructions, and the stored instructions may be executed by the processor 801 to implement the various functions described above. The working memory 802 may comprise both a volatile memory and a non-volatile memory (e.g. RAM, ROM, etc.). The storage device 807 may comprise a hard disk drive, solid state drive, removable media, including external and removable drives, memory cards, flash memory, floppy disks, optical disks (e.g. CD, DVD), storage arrays, network attached storage, storage area networks, etc. The working memory 802 and storage device 807 may both be collectively referred to as memory or computer-readable storage media herein, and may be non-transitory media capable of storing computer-readable, processor-executable program instructions as computer program code, which may be executed by the processor 801 as a specific device configured to implement the operations and functions described in the examples herein.

According to some embodiments, the processor 801 may perform control and scheduling of at least one of the image processing circuit 803 and various other apparatuses and circuits comprised in the electronic device 800. According to some embodiments, at least some of the constituent parts shown in Fig. 8 may be connected to and/or communicate with each other via a bus 808.

Software elements (programs) may be located in the working memory 802, including but not limited to an operating system 802a, one or more application program 802b, a driver and/or other data and code.

According to some embodiments, instructions for performing the abovementioned control and scheduling may be comprised in the operating system 802a or one or more application program 802b.

According to some embodiments, instructions for executing the method steps of the present disclosure may be included in the one or more application program 802b, and the modules of the electronic device 800 mentioned above may be realized by the processor 801 reading and executing the instructions of the one or more application program 802b. In other words, the electronic device 800 may comprise the processor 801 and a memory storing a program (e.g. the working memory 802 and/or storage device 807), the program comprising instructions which, when executed by the processor 801, cause the processor 801 to perform the method according to various embodiments of the present disclosure.

According to some embodiments, some or all of the operations performed by the image processing circuit 803 may be realized by the processor 801 reading and executing the instructions of the one or more application program 802b.

Executable code or source code of instructions of software elements (programs) may be stored in a non-transitory computer-readable storage medium (e.g. the storage device 807), and when executed, may be stored in the working memory 802 (possibly compiled and/or installed). Thus, the present disclosure provides a computer-readable storage medium storing a program, the program comprising instructions which, when executed by a processor of an electronic device, cause the electronic device to perform the method according to various embodiments of the present disclosure. According to another embodiment, executable code or source code of instructions of software elements (programs) may also be downloaded from a remote location.

It should also be understood that various changes in form may be carried out according to particular requirements. For example, it is also possible to use custom hardware, and/or it is possible to use hardware, software, firmware, middleware, microcode, hardware description language or any combination thereof to realize each circuit, unit, module or element. For example, some or all of the circuits, units, modules or elements contained in the disclosed methods and devices may be realized by using assembly language or hardware programming language (such as VERILOG, VHDL, C++) to program hardware (e.g. a programmable logic circuit comprising a field programmable gate array (FPGA) and/or a programmable logic array (PLA)), using logic and algorithms according to the present disclosure.

According to some embodiments, the processor 801 in the electronic device 800 may be distributed on a network. For example, one processor may be used to perform some processing, and at the same time, other processing may be performed by another processor remote from said one processor. Other modules of the electronic device 800 may also be similarly distributed. Thus, the electronic device 800 may be interpreted as being a distributed computing system that performs processing at multiple locations. The processor 801 of the electronic device 800 may also be a processor of a cloud computing system, or a processor integrated with a blockchain.

Although embodiments or examples of the present disclosure have already been described with reference to the drawings, it should be understood that the abovementioned methods, systems and devices are merely exemplary embodiments or examples, and the scope of the present invention is not limited by these embodiments or examples, instead being defined solely by the granted claims and the equivalent scope thereof. Various key elements in the embodiments or examples may be omitted or may be replaced by equivalent key elements thereof. In addition, the steps may be executed in an order different from that described in the present disclosure. Furthermore, various key elements in the embodiments or examples may be combined in various ways. Importantly, as technology evolves, many key elements described here may be replaced by equivalent key elements appearing after the present disclosure.

## Claims

1. An X-ray dose adjustment method for a medical imaging system, the method comprising:
acquiring a first image which is captured by the medical imaging system and comprises a region to be examined;
subjecting the first image to image segmentation on the basis of a grayscale value of the first image to acquire a first sub-region and a second sub-region, wherein a grayscale value of the first sub-region is greater than a grayscale value of the second sub-region;
determining at least one of the first sub-region and the second sub-region to be a target region on the basis of a comparison of the grayscale value of the first sub-region with a first grayscale threshold; and
performing dose adjustment on the basis of the target region.

2. The method as claimed in claim 1, wherein the step of determining at least one of the first sub-region and the second sub-region to be a target region, on the basis of a comparison of the grayscale value of the first sub-region with a first grayscale threshold, comprises:
comparing a first average grayscale value of the first sub-region with the first grayscale threshold; and
in response to the first average grayscale value being greater than the first grayscale threshold, determining the second sub-region to be the target region.

3. The method as claimed in claim 1, wherein the step of determining at least one of the first sub-region and the second sub-region to be a target region, on the basis of a comparison of the grayscale value of the first sub-region with a first grayscale threshold, comprises:
comparing a first average grayscale value of the first sub-region with the first grayscale threshold; and
in response to the first average grayscale value being less than the first grayscale threshold, determining both the first sub-region and the second sub-region to be the target region.

4. The method as claimed in claim 2 or 3, further comprising:
acquiring a maximum grayscale value in the first image,
wherein the first grayscale threshold is set within the range of 80% - 90% of the maximum grayscale value.

5. The method as claimed in any one of claims 1 - 3, wherein the step of subjecting the first image to image segmentation on the basis of a grayscale value of the first image, to acquire a first sub-region and a second sub-region, comprises:
determining a second grayscale threshold on the basis of the grayscale value of the first image, wherein in the first image, a pixel with a grayscale value greater than the second grayscale threshold is segmented into the first sub-region, and a pixel with a grayscale value less than the second grayscale threshold is segmented into the second sub-region, wherein the determined second grayscale threshold is such that the variance between a first average grayscale value of the first sub-region and a second average grayscale value of the second sub-region is maximized.

6. The method as claimed in any one of claims 1 - 3, wherein the medical imaging system comprises a collimator for limiting an X-ray irradiation range, and wherein the step of acquiring a first image, which is captured by the medical imaging system and comprises a region to be examined, comprises:
acquiring an original image captured by the medical imaging system, the original image comprising the region to be examined and a collimator region; and
removing the collimator region from the original image to acquire the first image.

7. The method as claimed in claim 6, wherein the step of removing the collimator region from the original image, to acquire the first image, comprises:
acquiring current coordinates of the collimator in the medical imaging system;
determining an image contour corresponding to the collimator region in the original image according to the current coordinates of the collimator;
determining a contour, formed by extension by a predetermined distance from a side of the image contour remote from the collimator region, to be a removal boundary; and
removing from the original image a region defined outside the removal boundary, so as to acquire the first image.

8. The method as claimed in any one of claims 1 - 3, wherein the step of acquiring a first image, which is captured by the medical imaging system and comprises a region to be examined, comprises:
acquiring an original image captured by the medical imaging system, the original image comprising the region to be examined;
determining an actual dose of X-rays emitted by the medical imaging system, according to a preset region, on the basis of the original image;
comparing the actual dose with a target dose; and
in response to the difference between the actual dose and the target dose being within a preset range, acquiring the first image on the basis of the original image.

9. The method as claimed in claim 8, wherein the step of acquiring the first image on the basis of the original image comprises:
filtering the original image, to acquire the first image with image noise points removed therefrom.

10. The method as claimed in claim 8, further comprising:
in response to the difference between the actual dose and the target dose being greater than a maximum value of the preset range, performing dose adjustment on the basis of the original image.

11. The method as claimed in any one of claims 1 - 3, wherein the step of performing dose adjustment on the basis of the target region comprises:
determining a deviation between an actual dose of X-rays emitted by the medical imaging system and a target dose, on the basis of the target region; and
computing an updated ray exposure parameter for exposure of the next image, on the basis of the deviation.

12. A dose adjustment apparatus for a medical imaging system, the apparatus comprising:
a first image acquisition unit, configured to acquire a first image which is captured by the medical imaging system and comprises a region to be examined;
an image segmentation unit, configured to subject the first image to image segmentation on the basis of a grayscale value of the first image to acquire a first sub-region and a second sub-region, wherein a grayscale value of the first sub-region is greater than a grayscale value of the second sub-region;
a target region determining unit, configured to determine at least one of the first sub-region and the second sub-region to be a target region on the basis of a comparison of the grayscale value of the first sub-region with a first grayscale threshold; and
an exposure control unit, configured to perform dose adjustment on the basis of the target region.

13. A medical imaging system, comprising:
at least one processor; and
a memory in communicative connection with the at least one processor, wherein
the memory stores a computer program which, when executed by the at least one processor, realizes the method as claimed in any one of claims 1 - 11.
